**Europäisches Patentamt**

**European Patent Office**

**Office européen des brevets**

(19)

(11) Veröffentlichungsnummer: **0 175 096**
**B1**

(12) **EUROPÄISCHE PATENTSCHRIFT**

(45) Veröffentlichungstag der Patentschrift:
**07.12.88**

(21) Anmeldenummer: **85109361.7**

(22) Anmeldetag: **25.07.85**

(51) Int. Cl.⁴: **A 61 M 1/00,** A 61 B 17/22

(54) Vorrichtung zum Entfernen von Festkörpern oder Ablagerungen aus Körpergefässen.

(30) Priorität: **06.09.84 DE 8426270 U**

(43) Veröffentlichungstag der Anmeldung:
**26.03.86 Patentblatt 86/13**

(45) Bekanntmachung des Hinweises auf die Patenterteilung:
**07.12.88 Patentblatt 88/49**

(84) Benannte Vertragsstaaten:
**AT BE CH DE FR GB IT LI LU NL SE**

(56) Entgegenhaltungen:
**DE-A- 2 230 283**
**DE-A- 2 447 513**
**DE-A- 3 029 042**
**US-A- 3 542 031**

(73) Patentinhaber: **Veltrup, Elmar M., Dipl.-Ing.,
Hermann-Schumacher-Strasse 16a, D-4150 Krefeld (DE)**

(72) Erfinder: **Veltrup, Elmar M., Dipl.-Ing.,
Hermann-Schumacher-Strasse 16a, D-4150 Krefeld (DE)**

(74) Vertreter: **Stark, Walter, Dr.-Ing., Moerser Strasse 140,
D-4150 Krefeld (DE)**

ACTORUM AG

## Beschreibung

Die Erfindung betrifft eine Vorrichtung zum Entfernen von Festkörpern oder Ablagerungen aus Körpergefässen, bestehend aus einem Katheter mit einem Saugkanal und einem Druckkanal, der im Bereich der Öffnung des Saugkanals in eine Düse mündet, die in den Saugkanal und im wesentlichen parallel zur Achse des Saugkanals gerichtet ist.

Bei Ablagerungen kann es sich um Blutthromben handeln, die üblicherweise mit einem Lösungsmittel (Lyse) angeweicht und aufgelöst werden. Die aufgelösten Blutthromben werden vom Blutstrom weggespült. Es verbleiben allerdings Kalkreste, Bindegewebsstrukturen und Gerinnungsklümpchen, die nach wie vor gefährlich sind und deren Entfernung grosse Schwierigkeiten bereitet. Andere Festkörper oder Ablagerungen, wie Nierensteine, Gallensteine oder Kalksteine, versucht man auch mit einem Lösungsmittel, anzuweichen, damit sie sich auflösen. In schwierigen Fällen versucht man, diese Steine mit Ultraschall zu zertrümmern, damit die Trümmer auf natürliche Weise abgehen können. Auch dann können aber störende Reste verbleiben.

Eine Vorrichtung der eingangs beschriebenen Gattung ist aus der US-A-35 42 031 bekannt. Sie dient zum Entfernen von Uterusablagerungen. Der Saugkanal endet an einer seitlich am Katheter angebrachten Öffnung. Über den Druckkanal wird eine Spülflüssigkeit zugeführt, die den Transport der abgesaugten Ablagerungen im Saugkanal unterstützen soll. Mit dieser Vorrichtung sollen keine Festkörper entfernt werden.

Aufgabe der Erfindung ist es, die vollständige Entfernung von Festkörpern oder Ablagerungen aus Körpergefässen zu ermöglichen.

Diese Aufgabe wird mit einer Vorrichtung der eingangs beschriebenen Gattung dadurch gelöst, dass der Druckkanal am freien Ende des Katheters sich mit einer die Düse aufweisenden Zunge bis über die Öffnung des Saugkanals erstreckt, dass die Düse vor der Öffnung des Saugkanals angeordnet ist, und dass beide Kanäle durch düsenartige Öffnungen miteinander verbunden sind, die so ausgerichtet sind, dass die vom Druckkanal in den Saugkanal austretenden Strahlen den Transport im Saugkanal unterstützen.

Zum Entfernen von Festkörpern oder Ablagerungen aus den Körpergefässen wird der Katheter so ausgerichtet, dass ein Festkörper oder eine Ablagerung zwischen die Öffnung des Saugkanals und die Zunge des Druckkanals gebracht wird. Der Druckkanal wird an eine Hochdruckpumpe angeschlossen und der Saugkanal an eine Vakuumpumpe. Unter der Wirkung des aus der Düse in der Zunge austretenden Flüssigkeitsstrahls wird der Festkörper zertrümmert bzw. die Ablagerung aufgelöst. Die Trümmer werden dann unter der Wirkung des Unterdrucks im Saugkanal in diesen eingesaugt, wobei der Transport in und durch den Saugkanal durch die aus der Düse in der Zunge sowie aus den weiteren Öffnungen austretenden Flüssigkeitsstrahlen unterstützt wird.

Grundsätzlich kann man auf diese Weise auch andere Festkörper, wie Nierensteine, Gallensteine oder dergleichen entfernen, wobei diese Steine allerdings zunächst vor die Öffnung des Saugkanals und unter die Düse des Druckkanals gebracht werden müssen. Das ist leichter, wenn der Druckkanal mit seiner sich über die Öffnung des Saugkanals erstreckenden Zunge als Kanüle ausgebildet ist, die längsverschieblich in einem Führungskanal des Katheters angeordnet ist. Dann kann ein Stein zwischen Öffnung des Saugkanals und Zunge eingefangen und eingespannt werden. Unter der Wirkung des aus der Düse in der Zunge austretenden Flüssigkeitsstrahls wird der Stein zertrümmert. Die Trümmer werden dann abgesaugt. Grössere Trümmer können wieder eingefangen und weiter zerkleinert werden.

Wegen der geringen Abmessungen von Blutgefässen kann auch der Katheter selbst nur einen kleinen Durchmesser aufweisen. Für den Transport des abgesaugten Materials ist es zweckmässig, wenn der Querschnitt des Saugkanals grösser ist als der Querschnitt des Druckkanals. Dafür kann die durch den Druckkanal transportierte Flüssigkeit mit entsprechend hohem Druck gefördert werden, z.B. mit einem Druck von bis zu 30 bar. Selbst unter Berücksichtigung der Leitungsverluste zwischen Hochdruckpumpe und Düse am Ende des Katheters kann davon ausgegangen werden, dass die Druckflüssigkeit im Bereich der Düsen noch einen Druck von 5 bis 20 bar besitzt.

Um eine individuelle Anpassung der Strömungsverhältnisse im Bereich des Katheters zu ermöglichen, können Druckregler im Saugkanal und/oder im Druckkanal bzw. an der Vakuumpumpe und/oder der Hochdruckpumpe vorgesehen werden.

Schliesslich ist es auch möglich, das abgesaugte Gut optisch zu kontrollieren, wenn zwischen Saugkanal und Vakuumpumpe ein Schauglas für das abgesaugte Gut angeordnet ist.

Im folgenden wird ein in der Zeichnung dargestelltes Ausführungsbeispiel der Erfindung erläutert; es zeigen:

Fig. 1 in schematischer Darstellung eine Vorrichtung zum Absaugen von Blutthromben aus Blutgefässen,

Fig. 2 in vergrösserter Darstellung und teilweise einen Schnitt durch einen Katheter des Gegenstandes nach Fig. 1,

Fig. 3 eine Stirnansicht in Richtung des Pfeils III auf den Gegenstand nach Fig. 2.

Fig. 4 eine andere Ausführung des Gegenstandes nach Fig. 2.

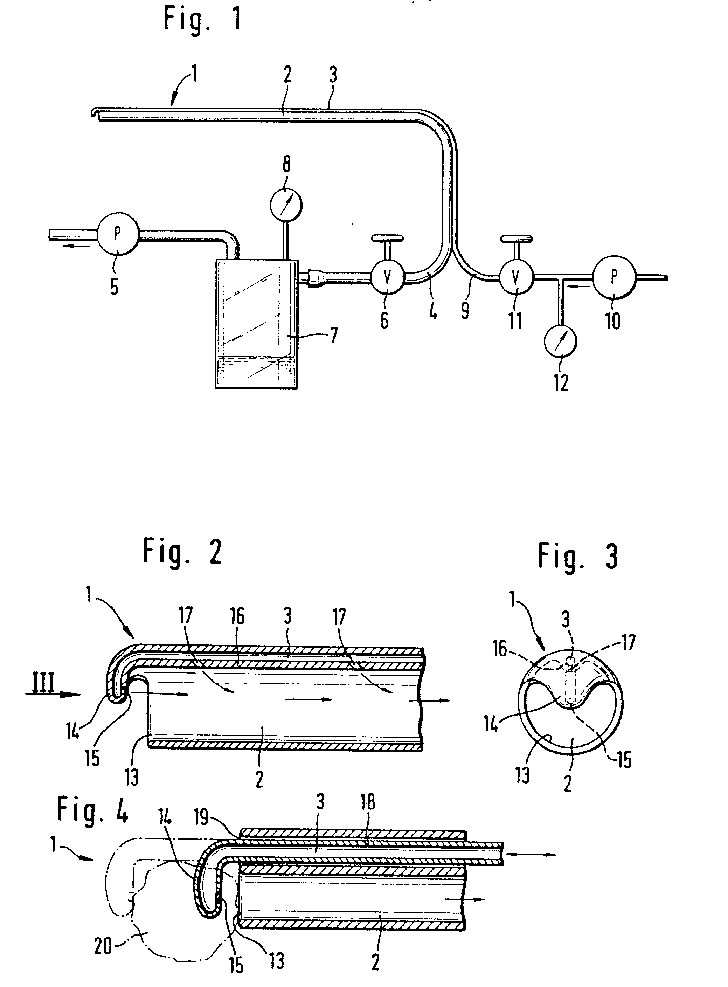

Zu der dargestellten Vorrichtung gehört ein Katheter 1 mit einem Saugkanal 2 und einem Druckkanal 3. Der Saugkanal 2 und der Druckkanal 3 sind zumindest im Bereich des in das Blutgefäss einführbaren Teilstücks des Katheters 1 einstückig ausgeführt, wie das in Fig. 2 dargestellt ist. Über dieses Teilstück hinaus können Saugkanal 2 und Druckkanal 3 einstückig oder als gesonderte Leitungen ausgebildet sein. Bei der Ausführung nach Fig. 1 schliesst an den Saugkanal 2 eine Saugleitung 4 an, die zu einer Vakuumpumpe 5 führt. In der Saugleitung 4 sind ein Druckregelventil 6 sowie ein Schauglas 7 mit einer Druckanzeige 8 angeordnet.

An den Druckkanal 3 ist eine Druckleitung 9 angeschlossen, die zu einer Hochdruckpumpe 10 führt. Die Hochdruckpumpe ist für einen Druck von ca. 30 bar ausgelegt. In der Druckleitung 9 befindet sich ein Druckregelventil 11 und eine Druckanzeige 12.

Aus einem Vergleich der Fig. 2 und 3 entnimmt man, dass im Bereich des in das Blutgefäss einzuführenden Teilstücks des Katheters 1 der Saugkanal 2 und der Druckkanal 3 parallel zueinander geführt sind. Der Querschnitt des Saugkanals 2 ist erheblich grösser als der Querschnitt des Druckkanals 3. Der Druckkanal 3 ist bis über die freie Öffnung 13 des Saugkanals 2 geführt und endet dort in einer diese Öffnung 13 teilweise abdeckenden Zunge 14. An der Unterseite der Zunge 14 befindet sich eine Düse 15, die so ausgerichtet ist, dass die daraus austretenden Strahlen im wesentlichen in den Saugkanal 2 gerichtet sind. Ausserdem sind in der gemeinsamen Wandung 16 zwischen Saugkanal 2 und Druckkanal 3 mehrere düsenartige Öffnungen 17, die bei der dargestellten Ausführung schräg zur Achse des Katheters so ausgerichtet sind, dass die daraus austretenden Strahlen den Transport im Saugkanal 2 unterstützen. Die Richtung der aus der Düse 15 bzw. den Öffnungen 17 austretenden Strahlen sind jeweils durch Pfeile angedeutet.

Die dargestellte Vorrichtung funktioniert wie folgt: der Katheter 1 wird in das Blutgefäss eingeschoben, bis sein vorderes Ende mit der Zunge 14 unmittelbar vor dem Thrombus liegt. Nach Einschalten der Vakuumpumpe 5 wird der Thrombus in die Öffnung 13 eingesaugt. Eine Verstopfung der Öffnung 13 bzw. des Saugkanals 2 wird verhindert, wenn die Hochdruckpumpe 10 eingeschaltet wird und aus der Düse 15 ein Flüssigkeitsstrahl austritt, der den Thrombus ganz oder in Teilen in den Saugkanal 2 hineintreibt. Beim Weitertransport des Thrombus oder seiner Teile durch den Saugkanal 2 treffen Teilstrahlen aus den düsenartigen Öffnungen 17 darauf, die nicht nur den Transport im Saugkanal 2 unterstützen sondern ggf. auch den Thrombus oder seine Teile weiterzerteilen.

Durch Verstellung der Druckregelventile 6 bzw. 11 können die Strömungsverhältnisse im Katheter den jeweiligen Umständen angepasst werden. Über das Schauglas 7 ist eine optische Kontrolle des abgesaugten Gutes möglich.

Bei der in Fig. 4 dargestellten Ausführung bezeichnen gleiche Bezugszeichen gleiche Teile. Dieser Katheter 1 ist insbesondere zum Entfernen von Nierensteinen, Gallensteinen und dergleichen geeignet. Sein Druckkanal 3 mit Zunge 14 besteht aus einer Kanüle 18, die in einem zugeordneten Führungskanal 19 des Katheters 1 längs verschieblich ist. Mit gestrichelten Linien ist die Kanüle 18 in ausgefahrener Stellung dargestellt, wobei zwischen der mit gestrichelten Linien dargestellten Zunge 14 und der Öffnung 13 des Saugkanals 2 ein mit gestrichelten Linien dargestellter Stein 20 gehalten ist. In der ausgefahrenen Stellung der Kanüle 18 kann das Ende des Katheters auch zum «Einfangen» eines Steins eingesetzt werden, der anschliessend in der dargestellten Weise eingespannt wird. Der aus der Düse 15 austretende Flüssigkeitsstrahl zertrümmert den Stein 20, dessen Trümmer abgesaugt werden. Grössere Trümmerstücke können wieder eingefangen und in der beschriebenen Weise zerkleinert und entfernt werden.

**Patentansprüche**

1. Vorrichtung zum Entfernen von Festkörpern oder Ablagerungen aus Körpergefässen, bestehend aus einem Katheter (1) mit einem Saugkanal (2) und einem Druckkanal (3), der im Bereich der Öffnung (13) des Saugkanals (2) in eine Düse (15) mündet, die in den Saugkanal (2) und im wesentlichen parallel zur Achse des Saugkanals (2) gerichtet ist, dadurch gekennzeichnet, dass der Druckkanal (3) am freien Ende des Katheters (1) sich mit einer die Düse (15) aufweisenden Zunge (14) bis über die Öffnung (13) des Saugkanals (2) erstreckt, dass die Düse (15) vor der Öffnung (13) des Saugkanals (2) angeordnet ist, und dass beide Kanäle (2, 3) durch düsenartige Öffnungen (17) miteinander verbunden sind, die so ausgerichtet sind, dass die vom Druckkanal in den Saugkanal austretenden Strahlen den Transport im Saugkanal (2) unterstützen.

2. Vorrichtung nach Anspruch 1, dadurch gekennzeichnet, dass der Druckkanal (3) mit seiner sich über die Öffnung (13) des Saugkanals (2) erstreckenden Zunge (14) als Kanüle (18) ausgebildet ist, die längsverschieblich in einem Führungskanal (19) des Katheters (1) angeordnet ist.

3. Vorrichtung nach einem der Ansprüche 1 oder 2, dadurch gekennzeichnet, dass der Querschnitt des Saugkanals (2) grösser ist als der Querschnitt des Druckkanals (3).

4. Vorrichtung nach einem der Ansprüche 1 bis 3, dadurch gekennzeichnet, dass der Saugkanal (2) an eine Vakuumpumpe (5) anschliessbar ist.

5. Vorrichtung nach Anspruch 4, dadurch gekennzeichnet, dass der Vakuumpumpe (5) ein Schauglas (7) für das abgesaugte Gut vorgeschaltet ist.

6. Vorrichtung nach einem der Ansprüche 1 bis 5, dadurch gekennzeichnet, dass der Druckkanal (3) an eine Hochdruckpumpe (10) anschliessbar ist.

7. Vorrichtung nach einem der Ansprüche 1 bis 6, gekennzeichnet durch Druckregler (6, 11) im Saugkanal (2) und/oder im Druckkanal (3).

**Revendications**

1. Dispositif pour débarrasser des vaisseaux corporels de corps solides ou de dépôts, consistant en un cathéter (1) qui comprend un canal d'aspiration (2) et un canal de pression (3) débouchant, au voisinage de l'ouverture (13) du canal d'aspiration (2), dans un ajutage (15) dirigé dans le canal d'aspiration (2), pour l'essentiel parallèlement à l'axe de ce canal d'aspiration (2), caractérisé par le fait que, à l'extrémité libre du cathéter (1), le canal de pression (3) s'étend jusqu'au-delà de l'ouverture (13) du canal d'aspiration (2), par une languette (14) qui présente l'ajutage (15); par le fait que l'ajutage (15) est disposé devant l'ouverture (13) du canal d'aspiration (2); et par le fait que les deux canaux (2, 3) sont reliés l'un à l'autre par l'intermédiaire d'orifices (17) du type ajutages qui sont orientés de telle sorte que les jets, parvenant dans le canal d'aspiration en provenance du canal de pression, accentuent le transport dans le canal d'aspiration (2).

2. Dispositif selon la revendication 1, caractérisé par le fait que le canal de pression (3) est réalisé, avec sa languette (14) dépassant de l'ouverture (13) du canal d'aspiration (2), sous la forme d'une canule (18) logée dans un canal de guidage (19) du cathéter (1), avec faculté de coulissement longitudinal.

3. Dispositif selon l'une des revendications 1 ou 2, caractérisé par le fait que la section du canal d'aspiration (2) est plus grande que la section du canal de pression (3).

4. Dispositif selon l'une des revendications 1 à 3, caractérisé par le fait que le canal d'aspiration (2) peut être raccordé à une pompe à vide (5).

5. Dispositif selon la revendication 4, caractérisé par le fait qu'un regard (7), destiné à la matière éliminée par aspiration, est installé en amont de la pompe à vide (5).

6. Dispositif selon l'une des revendications 1 à 5, caractérisé par le fait que le canal de pression (3) peut être raccordé à une pompe haute pression (10).

7. Dispositif selon l'une des revendications 1 à 6, caractérisé par des régulateurs de pression (6, 11) dans le canal d'aspiration (2) et/ou dans le canal de pression (3).

**Claims**

1. Apparatus for the removal of solid bodies or deposits from body vessels, comprising a catheter (1) having a suction duct (2) and a pressure duct (3), which opens out, in the region of the opening (13) of the suction duct (2), into a nozzle (15) which is directed into the suction duct (2) and essentially parallel to the axis of the suction duct (2), characterised in that the pressure duct (3) is extended at the free end of the catheter (1) over the opening (13) of the suction duct (2) by a tongue (14) having the nozzle (15), in that the nozzle (15) is arranged in front of the opening (13) of the suction duct (2), and in that both ducts (2, 3) are connected to one another by nozzle-like openings (17) which are arranged such that the jets emerging from the pressure duct into the suction duct assist transport in the suction duct (2).

2. Apparatus according to Claim 1, characterised in that the pressure duct (3), with its tongue (14) extending over the opening (13) of the suction duct (2), is constructed as a cannula (18) which is arranged so as to slide longitudinally in a guide duct (19) of the catheter (1).

3. Apparatus according to one of Claims 1 or 2, characterised in that the cross-section of the suction duct (2) is greater than the cross-section of the pressure duct (3).

4. Apparatus according to one of Claims 1 to 3, characterised in that the suction duct (2) can be connected to a vacuum pump (5).

5. Apparatus according to Claim 4, characterised in that a viewing glass (7) for the material which is removed by suction is placed in front of the vacuum pump (5).

6. Apparatus according to one of Claims 1 to 5, characterised in that the pressure duct (3) can be connected to a high-pressure pump (10).

7. Apparatus according to one of Claims 1 to 6, characterised by pressure regulators (6, 11) in the suction duct (2) and/or in the pressure duct (3).

# Fig. 1

1 2 3

8

P
5

V
6

4

9

V
11

P
10

12

7

# Fig. 2

1

17 16 3 17

III

14

15

13

2

# Fig. 3

1

3

16

17

14

15

13

2

# Fig. 4

1

14 19 3 18

20

15 13

2

5